# EUROPÄISCHE PATENTSCHRIFT

(11) **EP 2 944 619 B1**
(45) Veröffentlichungstag und Bekanntmachung des Hinweises auf die Patenterteilung: **09.08.2023**
(21) Anmeldenummer: 14168181.7
(22) Anmeldetag: 13.05.2014
(51) Int. Cl.: C03C 1/00, C09K 11/08, C03B 5/06, C03B 5/193, C03B 32/02, A61K 6/18, A61K 6/78

(54) **Verfahren zur Herstellung von Lithiumsilikatgläsern und Lithiumsilikat-Glaskeramiken**
Method for the preparation of lithium silicate glasses and lithium silicate glass ceramics
Procédé de fabrication de verres en silicate de lithium et vitrocéramique en silicate de lithium

(43) Veröffentlichungstag der Anmeldung: 18.11.2015
(73) Patentinhaber: Ivoclar Vivadent AG, 9494 Schaan (LI)
(72) Erfinder: Krolikowski, Sebastian, 8853 Lachen (CH); Rampf, Markus, 8853 Lachen (CH); Ritzberger, Christian, 9472 Grabs (CH); Bürke, Harald, 6820 Frastanz (AT); Höland, Wolfram, 9494 Schaan (LI); Schweiger, Marcel, 7000 Chur (CH)
(74) Vertreter: Uexküll & Stolberg

(56) Entgegenhaltungen:
- EP-A1- 0 916 625
- EP-A1- 1 688 398
- BEI ET AL: "Optical properties of Ce<3+>-doped oxide glasses and correlations with optical basicity", MATERIALS RESEARCH BULLETIN, ELSEVIER, KIDLINGTON, GB, Bd. 42, Nr. 7, 29. April 2007 (2007-04-29) , Seiten 1195-1200, XP022052727, ISSN: 0025-5408, DOI: 10.1016/J.MATERRESBULL.2006.10.020

## Beschreibung

Die vorliegende Erfindung betrifft ein Verfahren zur Herstellung eines Lithiumsilikatglases oder einer Lithiumsilikat-Glaskeramik, die Cer-Ionen enthalten und sich insbesondere zur Herstellung von Dentalrestaurationen eignen, deren Fluoreszenzeigenschaften weitgehend denen der natürlichen Zähne entsprechen. Die Erfindung betrifft auch ein Lithiumsilikatglas und eine Lithiumsilikat-Glaskeramik, die nach dem erfindungsgemäßen Verfahren erhältlich sind, deren Verwendung als Dentalmaterial und insbesondere zur Herstellung dentaler Restaurationen, sowie eine glasbildende Zusammensetzung, die zur Verwendung in dem erfindungsgemäßen Verfahren geeignet ist. Lithiumsilikat-Glaskeramiken werden in der Zahnheilkunde aufgrund ihrer hohen Transluzenz und sehr guten mechanischen Eigenschaften insbesondere zur Herstellung von Dentalkronen und kleinen Brücken eingesetzt. Die bekannten Lithiumsilikat-Glaskeramiken enthalten üblicherweise als Hauptkomponenten SiO₂, Li₂O, Al₂O₃, Na₂O oder K₂O und Keimbildner wie P₂O₅.

Die Dokumente EP 0 916 625 A1 und EP 1 688 398 A1 beschreiben transluzente Lithiumdisilikat-Glaskeramik-Produkte, die als Rohlinge herstellbar sind und insbesondere durch plastische Verformung unter Druck- und Wärmeeinwirkung oder spanende Bearbeitung zu geformten transluzenten Dentalprodukten mit hoher Festigkeit verarbeitet werden können. Zur Herstellung der Lithiumdisilikat-Glaskeramik-Produkte wird zunächst eine Schmelze eines Ausgangsglases erzeugt, welches die Komponenten SiO₂, Li₂O, La₂O₃ und/oder Al₂O₃ und MgO und/oder ZnO enthält. Diese Schmelze wird in geeigneter Weise geformt und abgekühlt und mindestens einer Wärmebehandlung unterzogen, um ein Glaskeramik-Produkt in Form eines Rohlings zu erhalten. Um die Farbe der Glaskeramik-Produkte an die Farbe des natürlichen Zahnmaterials anzupassen, kann das Ausgangsglas ferner Farb- und Fluoreszenzkomponenten aufweisen, welche vorzugsweise aus der Gruppe bestehend aus CeO₂, V₂O₅, Fe₂O₃, MnO₂, TiO₂, Y₂O₃, Er₂O₃, Tb₄O₇, Eu₂O₃, Yb₂O₃, Gd₂O₃, Nd₂O₃, Pr₂O₃, Dy₂O₃, Ag₂O, SnO₂ und Ta₂O₅ ausgewählt sind.

EP 1 505 041 A1 beschreibt Lithiummetasilikat-Glaskeramiken, die insbesondere mittels CAD/CAM-Verfahren zu Dentalrestaurationen verarbeitet werden und durch eine anschließende Wärmebehandlung in hochfeste Lithiumdisilikat-Glaskeramiken umgewandelt werden können. Zur Herstellung der Glaskeramiken wird zunächst eine Schmelze eines Ausgangsglases gebildet, das SiO₂, Li₂O, Al₂O₃, K₂O und einen Keimbildner wie P₂O₅ als Hauptkomponenten enthält. Die Schmelze des Ausgangsglases wird in geeigneter Weise geformt und abgekühlt und einer zweifachen Wärmebehandlung unterzogen, um ein Glaskeramik-Produkt in Form eines Rohlings zu erhalten. Das Ausgangsglas kann unter anderem farbgebende und fluoreszierende Metalloxide aufweisen. Dabei ist das Metall vorzugsweise aus der Gruppe bestehend aus Ta, Tb, Y, La, Er, Pr, Ce, Ti, V, Fe und Mn ausgewählt, wobei in den Beispielen die Oxide TiO₂, V₂O₅, Fe₂O₃, MnO₂, CeO₂, Y₂O₃, La₂O₃, Pr₂O₃, Ta₂O₅, Tb₄O₇ und Er₂O₃ eingesetzt werden. Ähnliche Lithiumsilikat-Glaskeramiken sind in EP 1 688 398 A1 beschrieben.

Aus W. Buchalla, "Comparative Fluorescence Spectroscopy Shows Differences in Non-Cavitated Enamel Lesions", Caries Res. 2005, 39, 150-156 ist bekannt, dass natürliche Zähne unter ultraviolettem Licht eine bläulich-weiße Fluoreszenz mit Wellenlängen im Bereich von 400 bis 650 nm zeigen.

Rukmani et al., J. Am. Ceram. Soc. 2007, 90, 706-711, beschreiben den Einfluss von V- und Mn-Färbemitteln auf das Kristallisationsverhalten und die optischen Eigenschaften von Ce-dotierten Lithiumdisilikat-Glaskeramiken. Zur Herstellung der Glaskeramiken wird eine Mischung aus den Ausgangsmaterialien SiO₂, ZrO₂, Li₂CO₃, K₂CO₃, MgCO₃ und Al(PO₃)₃ mit CeO₂, V₂O₅ und MnO₂ gemischt, die Mischung bei 1500°C in Platintiegeln geschmolzen, abgekühlt und anschließend in einem Röhrenofen unter Luftzufuhr mehreren Wärmebehandlungen unterzogen.

Der Artikel Bei et al. "Optical properties of Ce3+-doped oxide glasses and correlations with optical basicity", Materials Research Bulletin, Bd. 43, Nr. 7, 2007, Seiten 1195-1200 beschreibt die optischen Eigenschaften von Ce3+-dotierten Gläsern.

Es hat sich jedoch gezeigt, dass die aus dem Stand der Technik bekannten Lithiumsilikat-Glaskeramiken unzureichende Fluoreszenzeigenschaften aufweisen und insbesondere unter UV-Licht die Fluoreszenzeigenschaften des natürlichen Zahnmaterials nicht in ausreichendem Maße imitieren können. Dadurch werden aus solchen Glaskeramiken hergestellte Dentalrestaurationen insbesondere unter dem Einfluss von UV-Licht als Restaurationen erkennbar oder werden als Zahnlücken oder Defekte wahrgenommen.

Ausgehend von den oben beschriebenen Nachteilen der bereits bekannten Glaskeramiken liegt der Erfindung die Aufgabe zugrunde, eine Glaskeramik zur Verfügung zu stellen, die eine mit dem natürlichen Zahnmaterial vergleichbare Fluoreszenz zeigt und zur Herstellung von Dentalrestaurationen geeignet ist, welche die Farb- und Fluoreszenzeigenschaften des natürlichen Zahnmaterials insbesondere auch unter UV-Licht weitgehend imitieren können.

Diese Aufgabe wird erfindungsgemäß gelöst durch ein Verfahren zur Herstellung eines Lithiumsilikatglases oder einer Lithiumsilikat-Glaskeramik, welches einen Schritt umfasst, bei dem eine Schmelze eines Ausgangsglases, das Cer-Ionen enthält, reduzierenden Bedingungen ausgesetzt wird.

Es hat sich überraschend gezeigt, dass das erfindungsgemäße Verfahren die Herstellung von Lithiumsilikatgläsern und Lithiumsilikat-Glaskeramiken ermöglicht, die gegenüber dem Stand der Technik verbesserte Fluoreszenzeigenschaften insbesondere unter Einwirkung von UV-Licht zeigen.

Ohne Beschränkung auf eine bestimmte Theorie wird angenommen, dass es in Glasschmelzen, die Cer-Ionen enthalten, zur Einstellung eines Gleichgewichtes zwischen Ce³⁺-Ionen und Ce⁴⁺-Ionen kommt. Weiter wird davon ausgegangen, dass die reduzierenden Bedingungen, denen das Ausgangsglas in dem erfindungsgemäßen Verfahren ausgesetzt wird, dieses Verhältnis zugunsten von Ce³⁺-Ionen verschiebt, welche aufgrund von 5d→4f-Übergängen eine Fluoreszenz im Wellenlängenbereich von 320 bis 500 nm zeigen. Diese Fluoreszenz ist in besonderer Weise geeignet, die Fluoreszenzeigenschaften des natürlichen Zahnmaterials zu imitieren.

In der Regel beinhaltet das erfindungsgemäße Verfahren, dass die Schmelze des Ausgangsglases mit mindestens einem reduzierenden Mittel umgesetzt wird. Als reduzierende Mittel kommen grundsätzlich alle Mittel in Frage, die unter den Bedingungen des erfindungsgemäßen Verfahrens zur Reduktion von Ce⁴⁺-Ionen zu Ce³⁺-Ionen in der Lage sind. Dabei sind solche reduzierenden Mittel bevorzugt, die nach der Reduktion rückstandsfrei aus der Glasschmelze entfernt werden können.

Insbesondere sind gasförmige reduzierende Mittel sowie reduzierende Mittel bevorzugt, die unter den Bedingungen des erfindungsgemäßen Verfahrens nach der Reduktion aus der Glasschmelze ausgebrannt werden. Beispiele für gasförmige reduzierende Mittel sind Gase, die Wasserstoff und bevorzugt Mischungen von Wasserstoff und Stickstoff enthalten. Beispiele für reduzierende Mittel sind ferner Stoffe, die mindestens ein oxidierbares Kohlenstoffatom enthalten, insbesondere Kohlenstoff, beispielsweise Graphit, organische Salze, Kohlenhydrate und Getreidemehle.

Gemäß einer bevorzugten Ausführungsform wird die Schmelze des Ausgangsglases aus einer glasbildenden Zusammensetzung gebildet, die SiO₂, Li₂O, Keimbildner, eine Cerverbindung und mindestens ein reduzierendes Mittel enthält. Dabei ist als das mindestens eine reduzierende Mittel eine Verbindung bevorzugt, die mindestens ein oxidierbares Kohlenstoffatom enthält und vorzugsweise aus der Gruppe bestehend aus organischen Salzen, Kohlenhydraten und Getreidemehlen ausgewählt ist. Beispiele für besonders geeignete organische Salze sind Acetylacetonate.

In einer besonders bevorzugten Ausführungsform wird als reduzierendes Mittel ein Ceracetylacetonat, insbesondere Cer(III)-acetylacetonat verwendet. Gemäß dieser Ausführungsform stellt die Cerverbindung zugleich das mindestens eine reduzierende Mittel dar.

Gemäß einer weiteren bevorzugten Ausführungsform ist das mindestens eine reduzierende Mittel ein reduzierendes Gas, wobei das Gas vorzugsweise Wasserstoff enthält und bevorzugt Wasserstoff und Stickstoff enthält. Besonders geeignet sind Mischungen von Wasserstoff und Stickstoff, die etwa 5 Vol.-% Wasserstoff enthalten und auch als Formiergas bezeichnet werden. Dabei kann das Ausmaß der Reduktion über die Menge des zugeführten Gases und insbesondere über die Flussrate und Dauer der Zuführung des Gases gesteuert werden. Vorzugsweise beträgt die Menge der wirksamen Komponente des reduzierenden Gases, vorzugsweise Wasserstoff, 0,05 bis 5 l/min, insbesondere 0,1 bis 1 l/min und bevorzugt 0,2 bis 0,5 l/min, für eine Dauer von 10 bis 180 min, insbesondere 20 bis 120 min und bevorzugt 30 bis 90 min.

Erfindungsgemäß ist es bevorzugt, dass das Ausgangsglas 0,1 bis 7,0 Gew.-%, insbesondere 0,5 bis 5,0 Gew.-% und bevorzugt 1,0 bis 4,0 Gew.-% Cer-Ionen, berechnet als CeO₂, enthält.

Gemäß einer besonders bevorzugten Ausführungsform wird die Schmelze des Ausgangsglases aus einer glasbildenden Zusammensetzung gebildet, die mindestens eine Cer(III)-Verbindung und mindestens eine Cer(IV)-Verbindung enthält. Dabei kann durch die Variation des Verhältnisses von Cer(III)-Verbindung zu Cer(IV)-Verbindung das Verhältnis von Ce³⁺-Ionen und Ce⁴⁺-Ionen in dem erhaltenen Lithiumsilikatglas oder der Lithiumsilikat-Glaskeramik zusätzlich eingestellt werden. Zudem bewirken Ce⁴⁺-Ionen eine Gelbfärbung des Lithiumsilikatmaterials. Somit wird eine besonders gute Imitation der Fluoreszenz- und Farbeigenschaften des natürlichen Zahnmaterials ermöglicht. In einer besonders bevorzugten Ausführungsform enthält die glasbildende Zusammensetzung 0,1 bis 5,0 Gew.-%, insbesondere 0,5 bis 3,0 und bevorzugt 1,5 bis 2,0 Gew.-% Cer(III)-Verbindung, berechnet als Ce₂O₃, und 0,1 bis 5,0 Gew.-%, insbesondere 0,5 bis 3,0 und bevorzugt 1,5 bis 2,0 Gew.-% Cer(IV)-Verbindung, berechnet als CeO₂. Es ist weiter bevorzugt, dass das Massenverhältnis von Cer(III)-Verbindung, berechnet als Ce₂O₃, zu Cer(IV)-Verbindung, berechnet als CeO₂, im Bereich von 5:1 bis 1:5, insbesondere 2:1 bis 1:2 und bevorzugt 1,25:1 bis 1:1,25 liegt.

Das Ausgangsglas enthält ferner mindestens die zur Bildung einer Lithiumsilikat-Kristallphase erforderlichen Komponenten SiO₂, Li₂O und Keimbildner.

Bevorzugt enthält das Ausgangsglas 55,0 bis 75,0 Gew.-%, insbesondere 59,0 bis 73,0 Gew.-%, bevorzugt 60,0 bis 71,0 Gew.-% und besonders bevorzugt 60 bis 69 Gew.-% SiO₂.

Außerdem ist ein Ausgangsglas bevorzugt, das 9,0 bis 21,0 Gew.-%, insbesondere 13,0 bis 19,0 Gew.-% und bevorzugt 11,0 bis 15,0 Gew.-% Li₂O enthält.

Weiter hat es sich als besonders bevorzugt erwiesen, wenn das Ausgangsglas 0,5 bis 12,0 Gew.-% und insbesondere 2,5 bis 7,0 Gew.-% Keimbildner enthält. Bevorzugte Keimbildner sind P₂O₅, TiO₂, Nb₂O₅, Metalle, z.B. Pt, Pd, Au und Ag, und Mischungen davon. Besonders bevorzugt enthält das Ausgangsglas P₂O₅ als Keimbildner.

Das Ausgangsglas enthält bevorzugt weiteres Alkalimetalloxid in einer Menge von 1,0 bis 10,0 Gew.-%, insbesondere 1,0 bis 10,0 Gew.-%, bevorzugt 2,0 bis 7,0 Gew.-% und besonders bevorzugt 2,0 bis 5,0 Gew.-%. Der Begriff "weiteres Alkalimetalloxid" bezeichnet Alkalimetalloxid mit Ausnahme von Li₂O. Das weitere Alkalimetalloxid ist insbesondere Na₂O, K₂O, Cs₂O und/oder Rb₂O und ist besonders bevorzugt K₂O. Es ist bevorzugt, dass das Ausgangsglas weniger als 2,0 Gew.-%, insbesondere weniger als 1,0 Gew.-%, bevorzugt weniger als 0,5 Gew.-% und besonders bevorzugt im Wesentlichen kein Na₂O enthält.

Weiter ist es bevorzugt, dass das Ausgangsglas bis zu 5,0 Gew.-% Erdalkalimetalloxid enthält, wobei das Erdalkalimetalloxid insbesondere CaO, BaO, MgO, SrO oder eine Mischung davon ist.

Es ist weiter ein Ausgangsglas bevorzugt, das 0,5 bis 5,0, insbesondere 2,5 bis 7,0 und bevorzugt 2,5 bis 3,5 Gew.-% Oxid dreiwertiger Elemente enthält, wobei dieses Oxid insbesondere ausgewählt ist aus Al₂O₃, Y₂O₃, La₂O₃, Bi₂O₃ und Mischungen davon, und bevorzugt Al₂O₃ ist.

Besonders bevorzugt ist ein Ausgangsglas, das mindestens eine und bevorzugt alle der folgenden Komponenten enthält:

| Komponente | Gew.-% |
|---|---|
| SiO₂ | 55,0 bis 75,0, insbesondere 59,0 bis 73,0 |
| Li₂O | 9,0 bis 21,0, insbesondere 13,0 bis 19,0 |
| M₂O | 1,0 bis 12,0, insbesondere 2,0 bis 5,0 |
| Al₂O₃ | 0,5 bis 5,0, insbesondere 2,5 bis 3,5 |
| P₂O₅ | 0,5 bis 12,0, insbesondere 2,5 bis 7,0, |

wobei M₂O aus der Gruppe bestehend aus Na₂O, K₂O, Rb₂O und Cs₂O ausgewählt ist und vorzugsweise K₂O ist.

Das Ausgangsglas kann darüber hinaus noch Zusatzkomponenten enthalten, die insbesondere ausgewählt sind aus Oxiden vierwertiger Elemente, weiteren Oxiden fünfwertiger Elemente, Oxiden sechswertiger Elemente, Schmelzbeschleunigern sowie weiteren Färbemitteln und Fluoreszenzmitteln.

Der Begriff "weitere Oxide vierwertiger Elemente" bezeichnet Oxide vierwertiger Elemente mit Ausnahme von SiO₂. Beispiele für weitere Oxide vierwertiger Elemente sind ZrO₂, SnO₂ und GeO₂. In einer bevorzugten Ausführungsform enthält das Ausgangsglas 0,1 bis 15 Gew.-%, insbesondere 1 bis 10 Gew.-%, vorzugsweise 2 bis 8 Gew.-% und am meisten bevorzugt 4 bis 6 Gew.-% ZrO₂.

Der Begriff "weitere Oxide fünfwertiger Elemente" bezeichnet Oxide fünfwertiger Elemente mit Ausnahme von P₂O₅. Ein Beispiel für ein weiteres Oxid fünfwertiger Elemente ist Bi₂O₅.

Beispiele für Oxide sechswertiger Elemente sind WO₃ und MoO₃.

Bevorzugt ist eine Glaskeramik, die mindestens ein weiteres Oxid vierwertiger Elemente, ein weiteres Oxid fünfwertiger Elemente oder ein Oxid sechswertiger Elemente enthält.

Beispiele für Schmelzbeschleuniger sind Fluoride.

Beispiele für weitere Färbemittel und Fluoreszenzmittel sind Oxide von d- und f-Elementen, wie z.B. die Oxide von Sc, Ti, V, Mn, Fe, Ag, Ta, W, Pr, Nd, Eu, Gd, Tb, Dy, Er, Tm und Yb und insbesondere von V, Mn, Eu, Dy, Er und Tm.

In einer besonderen Ausführungsform enthält das Ausgangsglas ferner Terbium-Ionen. Bevorzugt enthält das Ausgangsglas 0,05 bis 2,0, insbesondere 0,1 bis 1,5, bevorzugt 0,2 bis 1,0 und besonders bevorzugt 0,3 bis 0,7 Gew.-% Terbium-Ionen, berechnet als Tb₄O₇. Es hat sich überraschend gezeigt, dass erfindungsgemäß durch Kombination von Cer-Ionen und Terbium-Ionen Lithiumsilikatgläser und Lithiumsilikat-Glaskeramiken erhalten werden können, deren Fluoreszenz- und Farbeigenschaften die des natürlichen Zahnmaterials besonders gut imitieren können. Besonders überraschend ist dabei, dass bei den erfindungsgemäß hergestellten Gläsern und Glaskeramiken die durch die Cer-Ionen hervorgerufene Fluoreszenz auch in Anwesenheit von Terbium-Ionen weitgehend bestehen bleibt, obwohl im Stand der Technik eine Verringerung oder gar Auslöschung der durch Cer-Ionen hervorgerufenen Fluoreszenz bei Anwesenheit von d-Elementen beobachtet wurde.

Die Bildung der Schmelze des Ausgangsglases erfolgt vorzugsweise bei Temperaturen von insbesondere 1300 bis 1600°C. Dabei wird insbesondere so vorgegangen, dass eine Mischung von geeigneten Ausgangsmaterialien, wie z.B. Carbonaten, Oxiden, Phosphaten und Fluoriden, bei Temperaturen von 1300 bis 1600°C für 2 bis 10 h erschmolzen wird. Im Falle der Verwendung eines Gases als reduzierendes Mittel wird die so erhaltene Glasschmelze mit dem Gas durchströmt. Anschließend kann die erhaltene Glasschmelze zur Erzielung einer besonders hohen Homogenität in Wasser gegossen werden, um ein Glasgranulat zu bilden, und das erhaltene Granulat dann erneut aufgeschmolzen werden.

Die Schmelze kann dann in Formen gegossen werden, um Rohlinge des Ausgangsglases, sogenannte Massivglasrohlinge oder monolithische Rohlinge, zu erzeugen. Es ist ebenfalls möglich, die Schmelze erneut in Wasser zu geben, um ein Granulat herzustellen. Dieses Granulat kann dann nach Mahlen und gegebenenfalls Zugabe weiterer Komponenten zu einem Rohling, einem sogenannten Pulverpressling, gepresst werden. Schließlich kann das Ausgangsglas nach Granulierung auch zu einem Pulver verarbeitet werden.

Anschließend kann das Ausgangsglas, z.B. in Form eines Massivglasrohlings, eines Pulverpresslings oder in Form eines Pulvers, mindestens einer Wärmebehandlung im Bereich von 450 bis 950°C unterzogen werden. Es ist bevorzugt, dass zunächst bei einer Temperatur im Bereich von 500 bis 600°C eine erste Wärmebehandlung durchgeführt wird, um ein Glas mit Keimen herzustellen, welche zur Bildung von Lithiummetasilikat- und/oder Lithiumdisilikat-kristallen geeignet sind. Dieses Glas kann dann bevorzugt mindestens einer weiteren Temperaturbehandlung bei einer höheren Temperatur und insbesondere mehr als 570°C unterworfen werden, um Kristallisation von Lithiummetasilikat oder von Lithiumdisilikat zu bewirken.

Der im Folgenden verwendete Begriff "Hauptkristallphase" bezeichnet die Kristallphase, die gegenüber anderen Kristallphasen den höchsten Volumenanteil hat.

Die nach dem erfindungsgemäßen Verfahren erhaltene Glaskeramik weist bevorzugt Lithiummetasilikat als Hauptkristallphase auf. In einer Ausführungsform enthält die Glaskeramik mehr als 10 Vol.-%, bevorzugt mehr als 20 Vol.-% und besonders bevorzugt mehr als 30 Vol.-% an Lithiummetasilikat-Kristallen, bezogen auf die gesamte Glaskeramik.

In einer weiteren bevorzugten Ausführungsform weist die Glaskeramik Lithiumdisilikat als Hauptkristallphase auf. In einer Ausführungsform enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik.

Die Erfindung betrifft ferner ein Lithiumsilikatglas, ein Lithiumsilikatglas mit zur Bildung von Lithiummetasilikat- und/oder Lithiumdisilikatkristallen geeigneten Keimen und eine Lithiumsilikat-Glaskeramik, die nach dem erfindungsgemäßen Verfahren erhältlich sind. Bevorzugte Ausführungsformen für das Lithiumsilikatglas, das Lithiumsilikatglas mit zur Bildung von Lithiummetasilikat- und/oder Lithiumdisilikatkristallen geeigneten Keimen und die Lithiumsilikat-Glaskeramik ergeben sich aus den oben für das erfindungsgemäße Verfahren beschriebenen bevorzugten Ausführungsformen.

Die Erfindung betrifft ferner ein Lithiumsilikatglas, ein Lithiumsilikatglas mit zur Bildung von Lithiummetasilikat- und/oder Lithiumdisilikatkristallen geeigneten Keimen und eine Lithiumsilikat-Glaskeramik, die bei einer Anregungswellenlänge von 366 nm eine Fluoreszenzintensität bei 430 nm und/oder im Wellenlängenbereich von 400 bis 460 nm (Fläche unter der Kurve) aufweisen, die mindestens das 1,5-fache, insbesondere mindestens das 2-fache, bevorzugt mindestens das 4-fache, besonders bevorzugt mindestens das 6-fache, der entsprechenden Fluoreszenzintensität einer Referenzprobe beträgt,
wobei die Referenzprobe dadurch erhältlich ist, dass ein Ausgangsglas mit der Zusammensetzung 71,3 Gew.-% SiO₂, 15,1 Gew.-% Li₂O, 3,2 Gew.-% K₂O, 3,5 Gew.-% Al₂O₃, 3,3 Gew.-% P₂O₅, 1,5 Gew.-% CeO₂ und 0,7 Gew.-% Tb₄O₇ im 200 g Maßstab aus geeigneten Rohstoffen in einem Platin-Rhodium-Tiegel bei 1500°C für 1 h erschmolzen wird, 30 g der Glasschmelze in eine vorgewärmte Form gegossen werden, um einen Glasblock zu erzeugen, und der Glasblock durch aufeinanderfolgende Temperaturbehandlungen bei 500°C für 10 min, 700°C für 20 min und 850°C für 10 min in eine Glaskeramik umgewandelt wird, wobei die Aufheizraten zwischen den Temperaturbehandlungen jeweils 30 K/min betragen.

Bevorzugt weisen das Lithiumsilikatglas und die Lithiumsilikat-Glaskeramik ferner bei einer Anregungswellenlänge von 366 nm eine Fluoreszenzintensität bei 541 nm und/oder im Wellenlängenbereich von 535 bis 555 nm (Fläche unter der Kurve) auf, die mindestens das 1,5-fache, insbesondere mindestens das 2-fache, bevorzugt mindestens das 3-fache, besonders bevorzugt mindestens das 4-fache, der entsprechenden Fluoreszenzintensität einer Referenzprobe beträgt, wobei die Referenzprobe wie oben beschrieben erhältlich ist.

Besonders bevorzugt sind Lithiumsilikatgläser und Lithiumsilikat-Glaskeramiken, die bei einer Anregungswellenlänge von 366 nm eine Fluoreszenzintensität im Wellenlängenbereich von 375 bis 700 nm (Fläche unter der Kurve) aufweisen, die mindestens das 1,5-fache, insbesondere mindestens das 2-fache, bevorzugt mindestens das 3-fache, besonders bevorzugt mindestens das 4-fache, der entsprechenden Fluoreszenzintensität einer Referenzprobe beträgt, wobei die Referenzprobe wie oben beschrieben erhältlich ist.

Die Messung der Fluoreszenz erfolgt typischerweise an Plättchen mit den Abmessungen 17.9 mm x 15.9 mm x 2 mm, deren Oberfläche mit einer APEX-Schleifscheibe (0,5 pm) poliert wurde, mittels eines Fluoreszenz-Spektrometers vom Typ FL1039 (Horiba Jobin Yvon GmbH) mit einer 450 W Xenon-Lampe, einem Anregungsmonochromator (Spaltbreite 1 nm, Anregungswellenlänge 366 nm), einem Emissionsmonochromator (Spaltbreite 1,5 nm, Scanbereich 372 bis 700 nm, Inkrement 1 nm) und einem Photomultiplier-Detektor (Integrationszeit 1 s) vom Typ PMT 1424M (Horiba Jobin Yvon GmbH). Das Plättchen wird dabei typischerweise in einem Winkel von 30° zum Anregungsmonochromator platziert und die Emission in einem Winkel von 90° zum Anregungsmonochromator mit einem optischen 5% Neutral Density Filter gemessen.

Die Erfindung betrifft ferner ein Lithiumsilikatglas, ein Lithiumsilikatglas mit zur Bildung von Lithiummetasilikat- und/oder Lithiumdisilikatkristallen geeigneten Keimen und eine Lithiumsilikat-Glaskeramik, die eine weißlich-blaue Fluoreszenzfarbe im CIE-Farbraum aufweisen.

Aus dem erfindungsgemäßen Lithiumsilikatglas, dem erfindungsgemäßen Lithiumsilikatglas mit zur Bildung von Lithiummetasilikat-und/oder Lithiumdisilikatkristallen geeigneten Keimen und der erfindungsgemäßen Lithiumsilikat-Glaskeramik können dentale Restaurationen, wie Inlays, Onlays, Veneers, Teilkronen, Kronen, Schalen oder Abutments hergestellt werden. Die Erfindung betrifft daher auch deren Verwendung als Dentalmaterial und insbesondere zur Herstellung dentaler Restaurationen.

Dabei ist es bevorzugt, dass die Glaskeramik oder das Glas durch Verpressen oder maschinelle Bearbeitung zur gewünschten dentalen Restauration verformt wird. Das Verpressen erfolgt üblicherweise unter erhöhtem Druck und erhöhter Temperatur. Es können für das Verpressen vor allem das erfindungsgemäße Lithiumsilikatglas und insbesondere das erfindungsgemäße Lithiumsilikatglas mit Keimen, die erfindungsgemäße Lithiummetasilikat- und die erfindungsgemäße Lithiumdisilikat-Glaskeramik in geeigneter Weise, z.B. in Form von Rohlingen, eingesetzt werden. Die maschinelle Bearbeitung wird üblicherweise im Rahmen eines CAD/CAM-Verfahrens durchgeführt, und sie nutzt insbesondere die erfindungsgemäße Lithiummetasilikat-und Lithiumdisilikat-Glaskeramik, vorzugsweise in Form von geeigneten Rohlingen. Nach der Herstellung der gewünscht geformten dentalen Restauration durch Verpressen oder maschinelle Bearbeitung kann diese insbesondere noch wärmebehandelt werden, um eingesetzte Vorläufer, wie Lithiumsilikatglas, Lithiumsilikatglas mit Keimen oder Lithiummetasilikat-Glaskeramik, in Lithiumdisilikat-Glaskeramik umzuwandeln.

Die Erfindung betrifft schließlich auch eine glasbildende Zusammensetzung, die SiO₂, Li₂O, Keimbildner, eine Cerverbindung und mindestens ein reduzierendes Mittel enthält. Diese Zusammensetzung eignet sich in besonderer Weise zum Einsatz in dem oben beschriebenen erfindungsgemäßen Verfahren. Bevorzugte Ausführungsformen der glasbildenden Zusammensetzung ergeben sich aus den oben für das erfindungsgemäße Verfahren beschriebenen bevorzugten Ausführungsformen. Die Erfindung wird im Folgenden anhand von Ausführungsbeispielen näher erläutert.

### Beispiele

Es wurden insgesamt 16 Gläser und Glaskeramiken mit den in der Tabelle I angegebenen Zusammensetzungen über Erschmelzung entsprechender Ausgangsgläser und anschließende Wärmebehandlung gemäß Tabelle II zur gesteuerten Keimbildung und Kristallisation hergestellt, wobei sich in Tabelle I die Oxidationsstufen der angegebenen Oxide auf die Oxidationsstufen der zur Erschmelzung der Ausgangsgläser eingesetzten Rohstoffe beziehen. In Tabelle II bedeuten
- T_{N} und t_{N}: angewendete Temperatur und Zeit für Keimbildung
- T_{K1} und t_{K1}: angewendete Temperatur und Zeit für erste Kristallisation
- T_{K2} und t_{K2}: angewendete Temperatur und Zeit für zweite Kristallisation.

### Beispiele 1 bis 10: Verwendung einer reduzierenden Cerverbindung als reduzierendes Mittel

Zur Herstellung von Gläsern und Glaskeramiken unter Verwendung einer Cerverbindung als reduzierendem Mittel wurden zunächst Ausgangsgläser entsprechend den in Tabelle I angegebenen Zusammensetzungen im 100 bis 200 g Maßstab aus einer Mischung üblicher Rohstoffe bei 1500°C für 2 h im Platintiegel erschmolzen, wobei als Rohstoff für den angegebenen Ce₂O₃-Gehalt Cer(III)-acetylacetonat eingesetzt wurde. Durch Eingießen der Ausgangsgläser in Wasser wurden Glasfritten hergestellt, die im Trockenschrank bei 150°C getrocknet und zur Homogenisierung anschließend ein zweites Mal bei 1500°C für 2,5 h geschmolzen wurden. Die erhaltenen Glasschmelzen wurden dann in vorgewärmte Formen gegossen, um Glasblöcke zu erzeugen.

Die Glasblöcke wurden dann durch thermische Behandlung zu Gläsern und Glaskeramiken umgewandelt. Die angewendeten thermischen Behandlungen zur gesteuerten Keimbildung und gesteuerten Kristallisation sind in Tabelle II angegeben.

### Beispiele 11 bis 15: Verwendung von Formiergas als reduzierendes Mittel

Zur Herstellung von Gläsern und Glaskeramiken unter Verwendung von Formiergas als reduzierendem Mittel wurden zunächst Ausgangsgläser entsprechend den in Tabelle I angegebenen Zusammensetzungen im 200 g Maßstab aus üblichen Rohstoffen in einem Platin-Rhodium-Tiegel bei 1450 bis 1500°C für 1 h erschmolzen. Anschließend wurden 30 g der Glasschmelzen als Referenzproben in vorgewärmte Formen gegossen, um Glasblöcke zu erzeugen. Die restliche Glasschmelze wurde für 30 bis 90 min mittels eines Quarzglas-Tauchrohrs mit etwa 3 l/min Formiergas (95% N₂, 5% H₂) durchströmt. Anschließend wurde das Tauchrohr aus der Schmelze entfernt und die Schmelzoberfläche für etwa 30 min mit Formiergas gespült, um eine Reoxidation zu vermeiden. Die Glasschmelze wurde anschließend in vorgewärmte Formen gegossen, um Glasblöcke zu erzeugen. Die nachfolgenden Temperaturbehandlungen (Keimbildung, Kristallisation und/oder Pressen) wurden in normaler Ofenatmosphäre durchgeführt.

Auswirkungen des Schmelzens unter Formiergas auf die Kristallisation und/oder Gefügeausbildung wurden nicht beobachtet.

### Beispiel 16: Verwendung einer organischen Verbindung als reduzierendem Mittel

Zur Herstellung von Gläsern und Glaskeramiken unter Verwendung einer organischen Verbindung als reduzierendem Mittel wurde ein Ausgangsglas entsprechend der in Tabelle I für Beispiel 11 angegebenen Zusammensetzung im 200 g Maßstab aus einer Mischung üblicher Rohstoffe unter Zusatz von 1,5 Gew.-% Saccharose im Platintiegel durch Aufheizen auf 1450°C mit einer Heizrate von 10 K/min erschmolzen. Nach einer Haltezeit von 30 min wurde die erhaltene Glasschmelze in Wasser gefrittet und anschließend getrocknet. Die Fritte wurde bei 1500°C erneut für 1 h aufgeschmolzen und in eine Graphitform gegossen, um Glasblöcke zu erzeugen.

Die Glasblöcke wurden dann durch thermische Behandlung zu Gläsern und Glaskeramiken umgewandelt. Hierzu wurden die Glasblöcke unmittelbar nach dem Giessen und Entformen in einem Muffelofen bei 490°C für 10 min getempert und anschließend langsam auf Raumtemperatur abgekühlt.

Von dem Glasblock wurde eine ca. 2 mm dicke Scheibe abgesägt und anschließend in einem Heizofen Programat (Ivoclar Vivadent AG) über eine Temperaturbehandlung bei 840°C für 7 min kristallisiert. Die so erhaltene weiße Lithiumdisilikatglaskeramik zeigte bei Anregung durch UV-Licht eine starke weiss-bläuliche Fluoreszenz.

Die Fluoreszenz dieser Probe ist im Vergleich zu einer herkömmlich erschmolzenen Glaskeramik stark erhöht und liegt im Bereich der Probe, welche mittels Durchleiten von Formiergas hergestellt wurde.

### Bestimmung von Biaxialfestigkeiten

Aus den nach Keimbildung und erster Kristallisation erhaltenen Blöcken wurden mit Hilfe einer Sirona-Schleifeinheit über das CAD/CAM-Verfahren Plättchen mit Dicken von ca. 2 mm herausgeschliffen. Die Plättchen wurden daraufhin in einem Heizofen Programat (Ivoclar Vivadent AG) für die zweite Kristallisation einer weiteren Temperaturbehandlung gemäß Tabelle II unterzogen. In einem weiteren Bearbeitungsschritt wurden die Plättchen auf eine Dicke von ca. 1,3 mm geschliffen und die Oberfläche mit einer Diamantschleifscheibe (15 pm) poliert. Die an den so erhaltenen Proben bestimmten mittleren Biaxialfestigkeiten sind in Tabelle II angegeben.

### Bestimmung von Farbwerten

Aus den nach Keimbildung und erster Kristallisation erhaltenen Blöcken wurden Scheiben von ca. 2,5 mm Dicke abgesägt und für die zweite Kristallisation einer weiteren Temperaturbehandlung gemäß Tabelle II unterzogen. Für die Bestimmung der Farbwerte wurden die Plättchen mit einem 1000er SiC-Schleifpapier auf eine Dicke von 2 mm geschliffen. Die Farbmesswerte wurden im Messbereich von 400-700 nm mittels eines Spektrophotometers CM-3700d (Konica-Minolta) gemessen. Dabei wurden die Farbwerte gemäß DIN5033 und DIN6174 und der CR-Wert gemäß British Standard BS56129 bestimmt.

### Fluoreszenzmessungen

Aus den nach Keimbildung und erster Kristallisation erhaltenen Blöcken wurden mit Hilfe einer Sirona-Schleifeinheit über das CAD/CAM-Verfahren Plättchen herausgeschliffen. Die Plättchen wurden daraufhin in einem Heizofen Programat (Ivoclar Vivadent AG) für die zweite Kristallisation einer weiteren Temperaturbehandlung gemäß Tabelle II unterzogen. In einem weiteren Bearbeitungsschritt wurden die Plättchen auf die Abmessungen 17.9 mm x 15.9 mm x 2 mm geschliffen und die Oberfläche mit einer APEX-Schleifscheibe (0,5 pm) poliert.

Zur Messung der Fluoreszenzeigenschaften wurde ein Fluoreszenz-Spektrometer vom Typ FL1039 (Horiba Jobin Yvon GmbH) mit einem Anregungsmonochromator und einem Emissionsmonochromator eingesetzt. Die Anregung der Proben erfolgte mittels einer 450 W Xenon-Lampe. Die Emissionsintensität wurde mit einem Photomultiplier-Detektor (PMT) vom Typ PMT 1424M (Horiba Jobin Yvon GmbH) als Impulse pro Sekunde (counts per second, cps) bestimmt. Die Kalibrierung des Anregungsmonochromators wurde mittels einer integrierten Silizium-Photodiode durchgeführt. Der Emissionsmonochromator wurde über die Position des Wasser-Ramanpeaks kalibriert. Die Linearität des Detektors im Messbereich wurde dabei über gerätespezifische Korrekturdateien sichergestellt. Die Linearität der Anregungsintensität wurde bei der Bestimmung der Anregungsspektren über eine mathematische Korrektur der gemessenen Emissionsintensität über die Lampenintensität (Division des gemessenen Signals durch das Referenzsignal der integrierten Silizium-Photodiode, welche direkt die Lampenintensität bestimmt) sichergestellt. Zum Schutz des Detektors und um nicht in den Sättigungsbereich zu gelangen, wurde im Emissionsstrahlengang ein 5% Neutral Density-Filter verwendet.

Die Proben wurden in einen Festkörperprobenhalter im Right-Angle-Modus eingespannt. Zur Vermeidung von Reflexionen des Anregungslichtes wurden die Proben um 30° zum Anregungsstrahl verdreht, so dass nur diffus gestreutes Emissionslicht detektiert wurde. Alle Proben wurden bei identischen Spektrometereinstellungen (Spaltbreiten 1 nm (Anregungsmonochromator) und 1,5 nm (Emissionsmonochromator), Scanbereich 372 bis 700 nm, Inkrement 1 nm, Integrationszeit 1 s, Anregungswellenlänge 366 nm) vermessen.

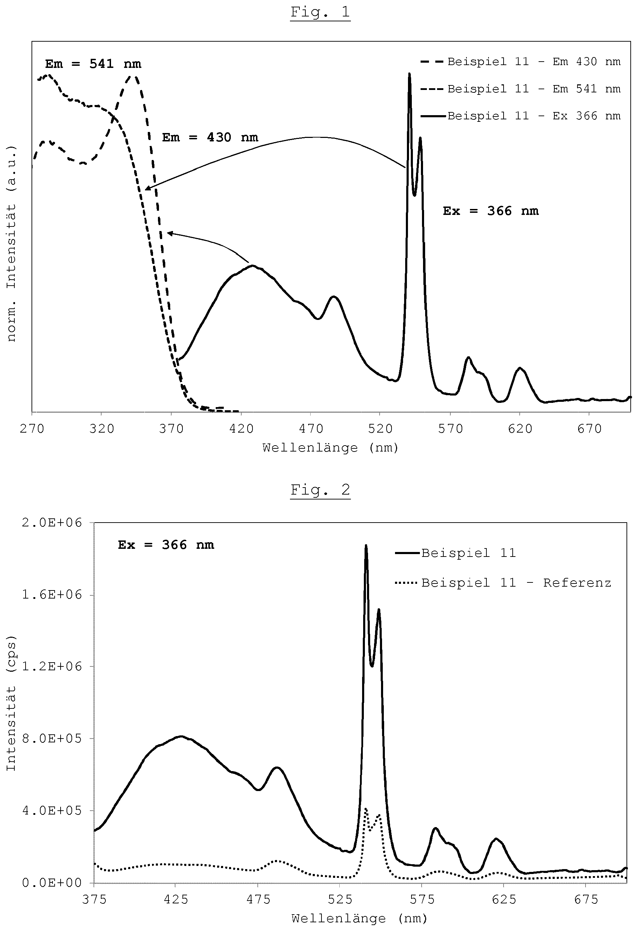

Fig. 1 zeigt für die gemäß Beispiel 11 erhaltene Glaskeramikprobe das Emissionsspektrum bei einer Anregungswellenlänge von 366 nm sowie Anregungsspektren für Emission bei 430 nm und 541 nm. Das Emissionsspektrum zeigte ein breites Maximum bei 430 nm, welches dem 5d --> 4f-Übergang von Ce³⁺ zuzuordnen ist. Das entsprechende Anregungsspektrum zeigte Anregungsmaxima bei 279 nm und 340 nm.

Ferner zeigte das Emissionsspektrum Maxima bei 483, 541, 585 und 619 nm welche den Übergängen ⁵D₄ → ⁷F₆, ⁷F₅, ⁷F₄ und ⁷F₃ von Tb³⁺ zuzuordnen sind. Das zugehörige breite Anregungsspektrum für Emission bei 541 nm zeigte Anregungsmaxima bei 279 nm und 315 nm. Die im Emissionsspektrum von Fig. 1 gezeigten Fluoreszenzemissionen werden vom menschlichen Auge insgesamt als weiss-blaue Fluoreszenz wahrgenommen.

Fig. 2 zeigt bei einer Anregungswellenlänge von 366 nm erhaltene Emissionsspektren für die Probe gemäß Beispiel 11, die unter reduzierenden Bedingungen mittels Durchströmen mit Formiergas hergestellt wurde, und die entsprechende Referenzprobe gleicher Zusammensetzung, die unter normalen Bedingungen an sauerstoffhaltiger Atmosphäre erschmolzen wurde. Erkennbar sind das breite Emissionsmaximum von Ce³⁺ bei etwa 430 nm und die Emissionsbanden von Tb³⁺ bei 483, 541, 549, 585 und 619 nm. Ein Vergleich der Spektren zeigt einen deutlichen Anstieg der Intensitäten der einzelnen Emissionsbanden durch das Schmelzen unter reduzierenden Bedingungen für die Probe gemäß Beispiel 11. Ein Vergleich der Gesamt-Lichtemission, bestimmt durch Berechnung des Flächenintegrals unter den Emissionskurven über den Bereich von 375 bis 700 nm (gesamter Messbereich), zeigt einen Anstieg um den Faktor 5,4.

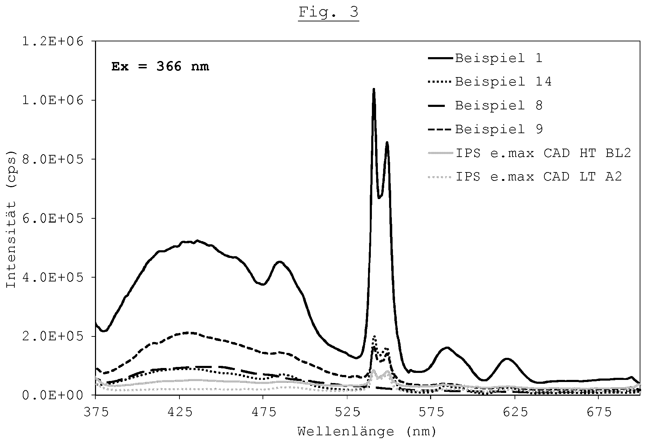

Fig. 3 zeigt die Emissionsintensitäten der Proben 1, 14, 8 und 9 im Vergleich zu den kommerziellen Lithiumsdisilikat-Glaskeramik-Produkten IPS e.max CAD HT BL2 und IPS e.max CAD LT A2 (Ivoclar Vivadent AG). Durch die jeweils verwendete Schmelztechnologie (Formiergas, reduzierender Rohstoff) ist es möglich, die Fluoreszenzintensität im Vergleich zu den kommerziellen Produkten deutlich zu steigern. So zeigte Beispiel 1 eine sehr starke Fluoreszenz, weshalb sich die Zusammensetzung insbesondere für die Verwendung als Abutment eignet. Die Beispiele 9 und 14 eignen sich auf Grund ihrer Farbe (unter Normbeleuchtung) und Fluoreszenz (unter UV-Licht) insbesondere für die Verwendung als Inlay und Kronenmaterial. Durch die Kombination von unterschiedlichen Cer-Rohstoffen (CeO₂, Ce(III)acac) wie im Falle von Beispiel 9 ist es möglich, eine intensive Farbwirkung unter D65-Normlicht und im Vergleich zu einem gleich gefärbten kommerziellen Produkt (IPS e.max CAD LT A2) eine deutlich gesteigerten Fluoreszenz zu erzeugen.

**Tabelle I**

| **Beispiel** | **1** | **2** | **3** | **4** | **5** | **6** | **7** | **8** | **9** | **10** | **11** | **12** | **13** | **14** | **15** |
|---|---|---|---|---|---|---|---|---|---|---|---|---|---|---|---|
| Zusammensetzung | Gew.% | Gew. % | Gew.% | Gew.% | Gew.% | Gew.% | Gew.% | Gew.% | Gew.% | Gew.% | Gew.% | Gew.% | Gew.% | Gew.% | Gew. % |
| SiO₂ | 69,1 | 68,0 | 68,0 | 59,0 | 69, 9 | 71, 9 | 70, 6 | 70,7 | 71,2 | 71,1 | 72,7 | 71,3 | 67,8 | 65,0 | 72,7 |
| GeO₂ | - | - | - | 2, 9 | - | - | - | - | - | - | - | - | - | - | - |
| ZrO₂ | - | - | 6, 0 | 6, 0 | - | - | - | - | - | - | - | 2,0 | 6, 0 | 9, 8 | - |
| Li₂O | 14,4 | 14,1 | 14,1 | 19,0 | 14,9 | 15,0 | 14,6 | 14,7 | 14,8 | 14,8 | 15,1 | 14,8 | 14,1 | 13,5 | 15,1 |
| P₂O₅ | 3,4 | 3,4 | 3,4 | 3,5 | 4,0 | 4,0 | 3,2 | 3,2 | 3,4 | 3,4 | 3,3 | 3,2 | 3,0 | 2,9 | 3,3 |
| Al₂O₃ | 3,4 | 3,3 | 3,0 | 3,0 | 3,0 | 3,0 | 3,4 | 3,4 | 3,4 | 3,4 | 3,5 | 3,4 | 3,2 | 3,1 | 3,5 |
| K₂O | - | - | 3,5 | 4,0 | 3,5 | 3,5 | 3,8 | 3,8 | 3,9 | 3,9 | 3,2 | 3,1 | 3,7 | 3,5 | 3,2 |
| Rb₂O | 7, 7 | - | - | - | - | - | - | - | - | - | - | - | - | - | - |
| Cs₂O | - | 9,2 | - | - | - | - | - | - | - | - | - | - | - | - | - |
| CaO | - | - | - | - | 2,0 | - | - | - | - | - | - | - | - | - | - |
| CeO₂ | - | - | - | - | - | - | 1,5 | 2,2 | 1,5 | 1,5 | 1,5 | 1,5 | 1,5 | 1,5 | 1,5 |
| Ce₂O₃* | 1,5 | 2,0 | 2,0 | 2,0 | 1,8 | 1,8 | 2,9 | 1,8 | 1,5 | 1,5 | - | - | - | - | - |
| Tb₄O₇ | 0,5 | - | - | 0,5 | 0,4 | 0,4 | - | - | - | - | 0,7 | 0,7 | 0,7 | 0,7 | - |
| Tb₂O₃ | - | - | - | - | - | - | - | - | 0,1 | 0,2 | - | - | - | - | - |
| Gd₂O₃ | - | - | - | - | - | 0,4 | - | - | - | - | - | - | - | - | - |
| Er₂O₃ | - | - | - | 0,1 | - | - | - | 0,1 | 0,1 | 0,1 | - | - | - | - | - |
| Eu₂O₃ | - | - | - | - | - | - | - | - | - | - | - | - | - | - | 0,7 |
| V₂O₅ | - | - | - | - | - | - | - | 0,1 | 0,1 | 0,1 | - | - | - | - | - |
| F | - | - | - | - | 0,5 | - | - | - | - | - | - | - | - | - | - |
| Σ | 100.0 | 100 | 100 | 100 | 100.0 | 100 | 100 | 100 | 100 | 100 | 100 | 100 | 100 | 100 | 100 |

| | | | | | | | | | | | | | | | |
|---|---|---|---|---|---|---|---|---|---|---|---|---|---|---|---|
| * eingesetzt als Cer(III)-acetylacetonat | | | | | | | | | | | | | | | |

**Tabelle II**

| | **1** | **2** | **3** | **4** | **5** | **6** | **7** | **8** | **9** | **10** | **11** | **12** | **13** | **14** | **15** |
|---|---|---|---|---|---|---|---|---|---|---|---|---|---|---|---|
| T_{N} [°C] | 520 | 560 | 530 | 500 | 500 | 510 | 470 | 490 | 490 | 490 | 500 | 500 | 500 | 500 | 500 |
| t_{N} [min] | 30 | 60 | 20 | 40 | 90 | 80 | 10 | 10 | 10 | 10 | 10 | 10 | 10 | 10 | 10 |
| T_{K1} [°C] | 700 | 700 | 700 | 700 | 700 | 700 | 700 | 700 | 700 | 700 | 700 | 700 | 650 | 650 | 700 |
| t_{K1} [min] | 20 | 20 | 20 | 20 | 20 | 20 | 20 | 20 | 20 | 40 | 20 | 20 | 20 | 20 | 20 |
| T_{K2} [°C] | 850 | 850 | 850 | 850 | 850 | 860 | 840 | 830 | 830 | 830 | 850 | 850 | 840 | 840 | 850 |
| t_{K2} [min] | 7 | 7 | 7 | 7 | 7 | 7 | 7 | 7 | 10 | 7 | 10 | 10 | 7 | 7 | 10 |
| | | | | | | | | | | | | | | | |
| σ_{B} [MPa] | 541,4 | | | | | | | | | | | | | | |
| L* | 93,37 | | | | | | | 79, 04 | 84, 02 | 81, 95 | | | 89, 08 | 86, 05 | |
| a* | 0,06 | | | | | | | 5, 61 | 2,54 | 3, 4 | | | 0, 14 | 1,29 | |
| b* | 4,37 | | | | | | | 25,03 | 18, 93 | 19, 69 | | | 12,22 | 10, 68 | |
| CR | 93, 37 | | | | | | | 78,38 | 79, 89 | 83,2 | | | 52,59 | 70,75 | |

## Patentansprüche

1. Verfahren zur Herstellung eines Lithiumsilikatglases oder einer Lithiumsilikat-Glaskeramik, welches einen Schritt umfasst, bei dem eine Schmelze eines Ausgangsglases, das Cer-Ionen enthält, reduzierenden Bedingungen ausgesetzt wird.

2. Verfahren nach Anspruch 1, bei dem die Schmelze des Ausgangsglases mit mindestens einem reduzierenden Mittel umgesetzt wird.

3. Verfahren nach Anspruch 2, bei dem die Schmelze des Ausgangsglases aus einer glasbildenden Zusammensetzung gebildet wird, die SiO₂, Li₂O, Keimbildner, eine Cerverbindung und mindestens ein reduzierendes Mittel enthält.

4. Verfahren nach Anspruch 2 oder 3, bei dem das mindestens eine reduzierende Mittel eine Verbindung ist, die mindestens ein oxidierbares Kohlenstoffatom enthält und vorzugsweise aus der Gruppe bestehend aus organischen Salzen, Kohlenhydraten und Getreidemehlen ausgewählt ist.

5. Verfahren nach Anspruch 4, bei dem das mindestens eine reduzierende Mittel ein Acetylacetonat, insbesondere ein Ceracetylacetonat und bevorzugt Cer(III)-acetylacetonat ist.

6. Verfahren nach Anspruch 2, bei dem das mindestens eine reduzierende Mittel ein reduzierendes Gas ist, wobei das Gas vorzugsweise Wasserstoff enthält und bevorzugt Wasserstoff und Stickstoff enthält.

7. Verfahren nach einem der Ansprüche 1 bis 6, bei dem das Ausgangsglas mindestens eine und bevorzugt alle der folgenden Komponenten enthält:
| Komponente | Gew.-% |
|---|---|
| SiO₂ | 55,0 bis 75,0, insbesondere 59,0 bis 73,0 |
| Li₂O | 9,0 bis 21,0, insbesondere 13,0 bis 19,0 |
| M₂O | 1,0 bis 12,0, insbesondere 2,0 bis 5,0 |
| Al₂O₃ | 0,5 bis 5,0, insbesondere 2,5 bis 3,5 |
| P₂O₅ | 0,5 bis 12,0, insbesondere 2,5 bis 7,0, |
wobei M₂O aus der Gruppe bestehend aus Na₂O, K₂O, Rb₂O und Cs₂O ausgewählt ist, insbesondere aus der Gruppe bestehend aus K₂O, Rb₂O und Cs₂O ausgewählt ist und vorzugsweise K₂O ist.

8. Verfahren nach einem der Ansprüche 1 bis 7, bei dem das Ausgangsglas ferner Terbium-Ionen enthält.

9. Verfahren nach einem der Ansprüche 1 bis 8 zur Herstellung eines Lithiumsilikatglases mit Keimen, die zur Ausbildung von Lithiummetasilikat- und/oder Lithiumdisilikatkristallen geeignet sind.

10. Verfahren nach einem der Ansprüche 1 bis 8 zur Herstellung einer Lithiumsilikat-Glaskeramik, die Lithiummetasilikat als Hauptkristallphase aufweist und/oder mehr als 10 Vol.-%, bevorzugt mehr als 20 Vol.-% und besonders bevorzugt mehr als 30 Vol.-% an Lithiummetasilikat-Kristallen aufweist.

11. Verfahren nach einem der Ansprüche 1 bis 8 zur Herstellung einer Lithiumsilikat-Glaskeramik, die Lithiumdisilikat als Hauptkristallphase aufweist und/oder mehr als 10 Vol.-%, bevorzugt mehr als 20 Vol.-% und besonders bevorzugt mehr als 30 Vol.-% an Lithiumdisilikat-Kristallen aufweist.

12. Verfahren nach einem der Ansprüche 1 bis 11, bei dem das Ausgangsglas mindestens einer Wärmebehandlung im Bereich von 450 bis 950°C unterzogen wird, um ein Lithiumsilikatglas mit Keimen, die zur Ausbildung von Lithiummetasilikat- und/oder Lithiumdisilikatkristallen geeignet sind, oder eine Lithiumsilikat-Glaskeramik zu bilden.

13. Verfahren nach einem der Ansprüche 1 bis 12, bei dem das Lithiumsilikatglas, das Lithiumsilikatglas mit zur Bildung von Lithiummetasilikat- und/oder Lithiumdisilikatkristallen geeigneten Keimen oder die Lithiumsilikat-Glaskeramik in Form eines Pulvers, eines Rohlings oder einer dentalen Restauration vorliegen.

14. Lithiumsilikatglas, Lithiumsilikatglas mit zur Bildung von Lithiummetasilikat- und/oder Lithiumdisilikatkristallen geeigneten Keimen oder Lithiumsilikat-Glaskeramik, die nach dem Verfahren gemäß einem der Ansprüche 1 bis 13 erhältlich sind.

15. Lithiumsilikatglas, Lithiumsilikatglas mit zur Bildung von Lithiummetasilikat- und/oder Lithiumdisilikatkristallen geeigneten Keimen oder Lithiumsilikat-Glaskeramik, die bei einer Anregungswellenlänge von 366 nm eine Fluoreszenzintensität bei 430 nm aufweisen, die mindestens das 1,5-fache, insbesondere mindestens das 2-fache, bevorzugt mindestens das 4-fache, besonders bevorzugt mindestens das 6-fache, der entsprechenden Fluoreszenzintensität einer Referenzprobe beträgt,
wobei die Referenzprobe dadurch erhältlich ist, dass ein Ausgangsglas mit der Zusammensetzung 71,3 Gew.-% SiO₂, 15,1 Gew.-% Li₂O, 3,2 Gew.-% K₂O, 3,5 Gew.-% Al₂O₃, 3,3 Gew.-% P₂O₅, 1,5 Gew.-% CeO₂ und 0,7 Gew.-% Tb₄O₇ im 200 g Maßstab aus geeigneten Rohstoffen in einem Platin-Rhodium-Tiegel bei 1500°C für 1 h erschmolzen wird, 30 g der Glasschmelze in eine vorgewärmte Form gegossen werden, um einen Glasblock zu erzeugen, und der Glasblock durch aufeinanderfolgende Temperaturbehandlungen bei 500°C für 10 min, 700°C für 20 min und 850°C für 10 min in eine Glaskeramik umgewandelt wird, wobei die Aufheizraten zwischen den Temperaturbehandlungen jeweils 30 K/min betragen.

16. Lithiumsilikatglas, Lithiumsilikatglas mit zur Bildung von Lithiummetasilikat- und/oder Lithiumdisilikatkristallen geeigneten Keimen oder Lithiumsilikat-Glaskeramik, die bei einer Anregungswellenlänge von 366 nm eine Fluoreszenzintensität bei 541 nm aufweisen, die mindestens das 1,5-fache, insbesondere mindestens das 2-fache, bevorzugt mindestens das 3-fache, besonders bevorzugt mindestens das 4-fache, der entsprechenden Fluoreszenzintensität einer Referenzprobe beträgt,
wobei die Referenzprobe wie in Anspruch 15 erhältlich ist.

17. Verwendung des Lithiumsilikatglases, des Lithiumsilikatglases mit zur Bildung von Lithiummetasilikat- und/oder Lithiumdisilikatkristallen geeigneten Keimen oder der Lithiumsilikat-Glaskeramik nach einem der Ansprüche 14 bis 16 als Dentalmaterial und insbesondere zur Herstellung dentaler Restaurationen.

18. Verwendung nach Anspruch 17, bei das Lithiumsilikatglas, das Lithiumsilikatglas mit zur Bildung von Lithiummetasilikat-und/oder Lithiumdisilikatkristallen geeigneten Keimen oder die Lithiumsilikat-Glaskeramik durch Verpressen oder maschinelle Bearbeitung zur gewünschten dentalen Restauration, insbesondere Inlay, Onlay, Veneer, Teilkrone, Krone oder Schale, verformt werden.

19. Glasbildende Zusammensetzung, die SiO₂, Li₂O, Keimbildner, eine Cerverbindung und mindestens ein reduzierendes Mittel enthält und vorzugsweise als Cerverbindung und reduzierendes Mittel eine Cerverbindung enthält, die mindestens ein oxidierbares Kohlenstoffatom enthält.

## Claims

1. Process for the preparation of a lithium silicate glass or a lithium silicate glass ceramic, which comprises a step in which a melt of a starting glass which comprises cerium ions is exposed to reducing conditions.

2. Process according to claim 1, in which the melt of the starting glass is reacted with at least one reducing agent.

3. Process according to claim 2, in which the melt of the starting glass is formed from a glass-forming composition which comprises SiO₂, Li₂O, nucleating agent, a cerium compound and at least one reducing agent.

4. Process according to claim 2 or 3, in which the at least one reducing agent is a compound which comprises at least one oxidizable carbon atom and is preferably selected from the group consisting of organic salts, carbohydrates and cereal flours.

5. Process according to claim 4, in which the at least one reducing agent is an acetylacetonate, in particular a cerium acetylacetonate and preferably cerium(III) acetylacetonate.

6. Process according to claim 2, in which the at least one reducing agent is a reducing gas, wherein the gas preferably comprises hydrogen and preferably comprises hydrogen and nitrogen.

7. Process according to any one of claims 1 to 6, in which the starting glass comprises at least one and preferably all of the following components:
| Component | wt.-% |
|---|---|
| SiO₂ | 55.0 to 75.0, in particular 59.0 to 73.0 |
| Li₂O | 9.0 to 21.0, in particular 13.0 to 19.0 |
| M₂O | 1.0 to 12.0, in particular 2.0 to 5.0 |
| Al₂O₃ | 0.5 to 5.0, in particular 2.5 to 3.5 |
| P₂O₅ | 0.5 to 12.0, in particular 2.5 to 7.0, |
wherein M₂O is selected from the group consisting of Na₂O, K₂O, Rb₂O and Cs₂O, in particular is selected from the group consisting of K₂O, Rb₂O and Cs₂O, and preferably is K₂O.

8. Process according to any one of claims 1 to 7, in which the starting glass furthermore comprises terbium ions.

9. Process according to any one of claims 1 to 8 for the preparation of a lithium silicate glass with nuclei which are suitable for forming lithium metasilicate and/or lithium disilicate crystals.

10. Process according to any one of claims 1 to 8 for the preparation of a lithium silicate glass ceramic which comprises lithium metasilicate as main crystal phase and/or comprises more than 10 vol.-%, preferably more than 20 vol.-% and particularly preferably more than 30 vol.-% lithium metasilicate crystals.

11. Process according to any one of claims 1 to 8 for the preparation of a lithium silicate glass ceramic which comprises lithium disilicate as main crystal phase and/or comprises more than 10 vol.-%, preferably more than 20 vol.-% and particularly preferably more than 30 vol.-% lithium disilicate crystals.

12. Process according to any one of claims 1 to 11, in which the starting glass is subjected to at least one heat treatment in the range of from 450 to 950°C in order to form a lithium silicate glass with nuclei which are suitable for forming lithium metasilicate and/or lithium disilicate crystals, or a lithium silicate glass ceramic.

13. Process according to any one of claims 1 to 12, in which the lithium silicate glass, the lithium silicate glass with nuclei suitable for forming lithium metasilicate and/or lithium disilicate crystals or the lithium silicate glass ceramic is present in the form of a powder, a blank or a dental restoration.

14. Lithium silicate glass, lithium silicate glass with nuclei suitable for forming lithium metasilicate and/or lithium disilicate crystals or lithium silicate glass ceramic, which can be obtained by the process according to any one of claims 1 to 13.

15. Lithium silicate glass, lithium silicate glass with nuclei suitable for forming lithium metasilicate and/or lithium disilicate crystals or lithium silicate glass ceramic, which at an excitation wavelength of 366 nm has a fluorescence intensity at 430 nm which is at least 1.5 times, in particular at least 2 times, preferably at least 4 times, particularly preferably at least 6 times, the corresponding fluorescence intensity of a reference sample,
wherein the reference sample is obtainable by melting a starting glass with the composition: 71.3 wt.-% SiO₂, 15.1 wt.-% Li₂O, 3.2 wt.-% K₂O, 3.5 wt.-% Al₂O₃, 3.3 wt.-% P₂O₅, 1.5 wt.-% CeO₂ and 0.7 wt.-% Tb₄O₇ on a scale of 200 g from suitable raw materials in a platinum-rhodium crucible at 1500°C for 1 h, pouring 30 g of the glass melt into a preheated mould to produce a glass block, and converting the glass block into a glass ceramic by successive temperature treatments at 500°C for 10 min, 700°C for 20 min and 850°C for 10 min, wherein the heating rates between the temperature treatments are 30 K/min in each case.

16. Lithium silicate glass, lithium silicate glass with nuclei suitable for forming lithium metasilicate and/or lithium disilicate crystals or lithium silicate glass ceramic, which at an excitation wavelength of 366 nm has a fluorescence intensity at 541 nm which is at least 1.5 times, in particular at least 2 times, preferably at least 3 times, particularly preferably at least 4 times, the corresponding fluorescence intensity of a reference sample,
wherein the reference sample is obtainable as in claim 15.

17. Use of the lithium silicate glass, of the lithium silicate glass with nuclei suitable for forming lithium metasilicate and/or lithium disilicate crystals or of the lithium silicate glass ceramic according to any one of claims 14 to 16 as dental material and in particular for the preparation of dental restorations.

18. Use according to claim 17, wherein the lithium silicate glass, the lithium silicate glass with nuclei suitable for forming lithium metasilicate and/or lithium disilicate crystals or the lithium silicate glass ceramic is shaped by pressing or machining to form the desired dental restoration, in particular inlay, onlay, veneer, partial crown, crown or facet.

19. Glass-forming composition which comprises SiO₂, Li₂O, nucleating agent, a cerium compound and at least one reducing agent and preferably comprises as cerium compound and reducing agent a cerium compound which comprises at least one oxidizable carbon atom.

## Revendications

1. Procédé de fabrication d'un verre de silicate de lithium ou d'une vitrocéramique de silicate de lithium, qui comprend une étape lors de laquelle un bain de fusion d'un verre de départ, contenant des ions de cérium, est exposé à des conditions réductrices.

2. Procédé selon la revendication 1, selon lequel le bain de fusion du verre de départ est réalisé avec au moins un agent réducteur.

3. Procédé selon la revendication 2, selon lequel le bain de fusion du verre de départ est réalisé à partir d'un composé formateur de verre qui contient du SiO₂, du Li₂O, des agents de nucléation, un composé au cérium et au moins un agent réducteur.

4. Procédé selon la revendication 2 ou 3, selon lequel l'agent réducteur, au nombre d'au moins un, est un composé qui contient au moins un atome de carbone oxydable et est de préférence choisi dans le groupe comprenant des sels organiques, des hydrates de carbone et des farines de céréales.

5. Procédé selon la revendication 4, selon lequel l'agent réducteur, au nombre d'au moins un, est un acétylacétonate, en particulier un acétylacétonate de cérium, et de préférence de l'acétylacétonate de Cer(III).

6. Procédé selon la revendication 2, selon lequel l'agent réducteur, au nombre d'au moins un, est un gaz réducteur, le gaz contenant de préférence de l'hydrogène et contenant de préférence de l'hydrogène et de l'azote.

7. Procédé selon l'une des revendications 1 à 6, selon lequel le verre de départ contient au moins un et de préférence tous les composants figurant ci-après :
| Composant | % en poids |
|---|---|
| SiO₂ | 55,0 à 75,0, notamment 59,0 à 73,0 |
| Li₂O | 9,0 à 21,0, notamment 13,0 à 19 ,0 |
| M₂O | 1,0 à 12,0, notamment 2,0 à 5 ,0 |
| Al₂O₃ | 0,5 à 5,0, notamment 2,5 à 3,5 |
| P₂O₅ | 0,5 à 12,0, notamment 2,5 à 7,0, |
où M₂O est choisi dans le groupe comprenant Na₂O, K₂O, Rb₂O et Cs₂O, en particulier dans le groupe comprenant K₂O, Rb₂O et Cs₂O, et est de préférence du K₂O.

8. Procédé selon l'une des revendications 1 à 7, selon lequel le verre de départ contient en outre des ions de terbium.

9. Procédé selon l'une des revendications 1 à 8, destiné à la fabrication d'un verre de silicate de lithium avec des germes qui conviennent pour la formation de cristaux de métasilicate de lithium et/ou de disilicate de lithium.

10. Procédé selon l'une des revendications 1 à 8, destiné à la fabrication d'une vitrocéramique de silicate de lithium qui présente du métasilicate de lithium en tant que phase cristalline principale et/ou présente plus de 10 % vol, de préférence plus de 20 % vol et de manière particulièrement avantageuse plus de 30 % vol de cristaux de métasilicate de lithium.

11. Procédé selon l'une des revendications 1 à 8, destiné à la fabrication d'une vitrocéramique de silicate de lithium qui présente du disilicate de lithium en tant que phase cristalline principale et/ou présente plus de 10 % vol, de préférence plus de 20 % vol et de manière particulièrement avantageuse plus de 30 % vol de cristaux de disilicate de lithium.

12. Procédé selon l'une des revendications 1 à 11, selon lequel le verre de départ est soumis à au moins un traitement thermique dans la plage allant de 450 à 950 °C pour former un verre de silicate de lithium avec des germes convenant pour la formation de cristaux de métasilicate de lithium et/ou de disilicate de lithium, ou une vitrocéramique de silicate de lithium.

13. Procédé selon l'une des revendications 1 à 12, selon lequel le verre de silicate de lithium, le verre de silicate de lithium avec des germes convenant pour la formation de cristaux de métasilicate de lithium et/ou de disilicate de lithium, ou la vitrocéramique de silicate de lithium sont présents sous la forme d'une poudre, d'une ébauche ou d'une restauration dentaire.

14. Verre de silicate de lithium, verre de silicate de lithium avec des germes convenant pour la formation de cristaux de métasilicate de lithium et/ou de disilicate de lithium, ou vitrocéramique de silicate de lithium, qui peuvent être obtenus avec le procédé selon l'une des revendications 1 à 13.

15. Verre de silicate de lithium, verre de silicate de lithium avec des germes convenant pour la formation de cristaux de métasilicate de lithium et/ou de disilicate de lithium, ou vitrocéramique de silicate de lithium, qui, en présence d'une longueur d'onde d'excitation de 366 nm, présentent une intensité de fluorescence de 430 nm qui vaut au moins 1,5 fois, en particulier au moins 2 fois, de préférence au moins 4 fois, et de manière particulièrement avantageuse au moins 6 fois l'intensité de fluorescence correspondante d'un échantillon de référence,
l'échantillon de référence pouvant être obtenu par le fait qu'un verre de départ, ayant la composition 71,3 % en poids SiO₂, 15,1 % en poids Li₂O, 3,2 % en poids K₂O, 3,5 % Al₂O₃, 3,3 % en poids P₂O₅, 1,5 % en poids CeO₂, et 0,7 % en poids Tb₄O₇, à l'échelle de 200 g, est mis en fusion à partir de matières premières appropriées dans un creuset en platine-rhodium, à 1 500 °C pendant 1 h, que 30 g du verre fondu sont versés dans un moule préalablement chauffé pour former un bloc de verre, et que le bloc de verre est transformé en une vitrocéramique par des traitements thermiques successifs à 500 °C pendant 10 min, 700 °C pendant 20 min et 850 °C pendant 10 min, les vitesses de chauffage entre les traitements thermiques étant chaque fois de 30 K/min.

16. Verre de silicate de lithium, verre de silicate de lithium avec des germes convenant pour la formation de cristaux de métasilicate de lithium et/ou de disilicate de lithium, ou vitrocéramique de silicate de lithium, qui, en présence d'une longueur d'onde d'excitation de 366 nm, présentent une intensité de fluorescence de 541 nm qui vaut au moins 1,5 fois, en particulier au moins 2 fois, de préférence au moins 3 fois, et de manière particulièrement avantageuse au moins 4 fois l'intensité de fluorescence correspondante d'un échantillon de référence,
l'échantillon de référence pouvant être obtenu de la manière indiquée dans la revendication 15.

17. Utilisation du verre de silicate de lithium, du verre de silicate de lithium avec des germes convenant pour la formation de cristaux de métasilicate de lithium et/ou de disilicate de lithium, ou de la vitrocéramique de silicate de lithium selon l'une des revendications 14 à 16, en tant que matériau dentaire et en particulier pour la fabrication de restaurations dentaires.

18. Utilisation selon la revendication 17, lors de laquelle le verre de silicate de lithium, le verre de silicate de lithium avec des germes convenant pour la formation de cristaux de métasilicate de lithium et/ou de disilicate de lithium, ou la vitrocéramique de silicate de lithium sont mis en forme par pressage ou usinage mécanique pour obtenir la restauration dentaire souhaitée, notamment de type inlay, onlay, facette dentaire, couronne partielle, couronne ou coque.

19. Composé formateur de verre qui contient du SiO₂, Li₂O, des agents de nucléation, un composé au cérium et au moins un agent réducteur et contient de préférence en tant que composé au cérium et agent réducteur, un composé au cérium qui comporte au moins un atome de carbone oxydable.
